# EUROPÄISCHE PATENTSCHRIFT

(11) **EP 0 266 612 B1**
(45) Veröffentlichungstag und Bekanntmachung des Hinweises auf die Patenterteilung: **28.04.1993**
(21) Anmeldenummer: 87115242.7
(22) Anmeldetag: 19.10.1987
(51) Int. Cl.: G01N 33/36

(54) **Vorrichtung zum automatischen Bestimmung von Kenngrössen von textilem Prüfgut**
Device for the automatic determination of data concerning a textile product
Appareil pour la détermination automatique de caractéristiques d'un produit textile

(30) Priorität: 07.11.1986 CH 4460/86
(43) Veröffentlichungstag der Anmeldung: 11.05.1988
(73) Patentinhaber: ZELLWEGER USTER AG, CH-8610 Uster (CH)
(72) Erfinder: Heusser, Eduard, CH-8610 Uster (CH)

(56) Entgegenhaltungen:
- FR-A- 2 169 461
- FR-A- 2 244 694

## Beschreibung

Die Erfindung betrifft eine Vorrichtung zur automatischen Bestimmung von Kenngrössen von textilem Prüfgut, wie Garnen, Vorgarnen und Bändern, mit einer eine Führungseinrichtung, ein Messorgan, eine Vorschubeinrichtung und ein Abzugsorgan für das Prüfgut enthaltenden Messeinheit, deren Vorschubeinrichtung durch ein Paar von Transportwalzen gebildet ist, längs deren Achsen beim Durchlaufen des Prüfguts eine Changierbewegung zwischen dem Prüfgut und den Transportwalzen erfolgt.

In den Labors von Textilbetrieben, vornehmlich von Spinnereien, werden im Rahmen der betrieblichen Qualitätskontrolle Stichprobenprüfungen zur Bestimmung gewisser textiler Parameter, wie beispielsweise der Masseschwankungen und anderer davon abgeleiteter Kenngrössen vorgenommen. Dazu dienen sogenannte Gleichmässigkeitsprüfer, wie sie beispielsweise von der Inhaberin des vorliegenden Patents unter der Bezeichnung USTER TESTER (USTER eingetragenes Warenzeichen der Zellweger Uster AG) weltweit vertrieben werden. Bei diesem bekannten Gleichmässigkeitsprüfer stellt jeder Messspalt den Luftspalt eines Kondensators dar, und die Bestimmung der genannten Kenngrössen erfolgt kapazitiv.

Zur Bestimmung der Masseschwankungen des Prüfguts wird dieses durch die Transportwalzen durch den Messkamm gezogen und nach der Prüfung durch das Abzugsorgan abgesaugt. Der Vorschub erfolgt dabei mit einer Geschwindigkeit von bis zu 400 m/min bei Prüfgut aus Stapelfasern und von bis zu 800 m/min bei Filamentgarnen. Es liegt auf der Hand, dass dabei dem einwandfreien Funktionieren der Transportwalzen eine wesentliche Bedeutung zukommt.

Ein den Vorschub des Prüfguts beeinflussender Faktor ist dabei die Abnützung der aus einem hartgummiartigen Material bestehenden Transportwalzen. Damit diese nicht immer an ein und derselben Stelle erfolgt, was zu Rillenbildung führen könnte, ist bei den bekannten Gleichmässigkeitsprüfern ein manuell betätigbarer Führungssteg vorgesehen, welcher von der Bedienungsperson vor jeder Prüfung verstellt werden soll, damit das Prüfgut den Spalt zwischen den Transportwalzen jeweils an einer anderen Stelle durchläuft. Abgesehen davon, dass auch die vorgeschriebene Verstellung des Führungsstegs keine gleichmässige Abnützung der Transportwalzen garantiert, wird diese Verstellung vom Bedienungspersonal auch nicht regelmässig vorgenommen.

Auf einem anderen textilen Fachgebiet, der Spulerei, ist es bekannt, siehe dazu beispielsweise die FR-A-2 169 461, den aufzuspulenden Faden zwischen den Transportwalzen zu changieren, wobei diese Changierbewegung durch ein längs der Achsen der Transportwalzen hin- und herbewegtes gabelförmiges Fadenführungsorgan erfolgt.

Durch die Erfindung soll nun der bekannte Gleichmässigkeitsprüfer so verbessert werden, dass eine möglichst gleichmässige Abnützung der Transportwalzen erfolgt und unter allen Umständen ein einwandfreier Vorschub gewährleistet ist, und dass sich der Fadenlauf während des Messvorgangs nicht ändert.

Diese Aufgabe wird durch das im kennzeichnenden Teil von Anspruch 1 angegebene Merkmal gelöst.

Erfindungsgemäss vollführt also nicht das Prüfgut eine Changierbewegung, sondern die Transportwalzen werden quer zum Prüfgut hin- und hergeschoben. Dadurch bleibt der Fadenlauf während des Messvorgangs gleich und es ergibt sich eine gleichmässige Abnützung der Transportwalzen.

Im folgenden wird die Erfindung anhand eines Ausführungsbeispiels und der Zeichnungen näher dargestellt; es zeigen:
- Fig. 1: eine Perspektivdarstellung eines Gleichmässigkeitsprüfers zur Bestimmung der Masseschwankungen von Stapelfasergarnen,
- Fig. 2: eine Perspektivdarstellung der wesentlichen Teile der Vorschubeinrichtung der Messeinheit des Gleichmässigkeitsprüfers,
- Fig. 3: eine Frontansicht eines Teils der Messeinheit, und
- Fig. 4: eine Ansicht in Richtung des Pfeils IV von Fig. 3, bei weggelassener unterer Gehäuseplatte.

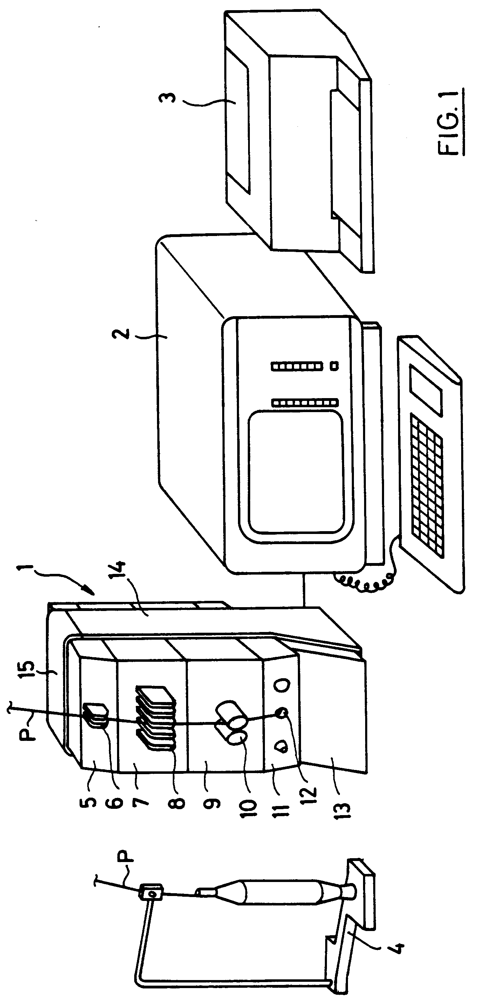

Der in Fig. 1 dargestellte Gleichmässigkeitsprüfer zur Bestimmung der Masseschwankungen von textilem Prüfgut, wie Garnen, Vorgarnen oder Bändern aus Stapelfasern, besteht darstellungsgemäss aus einer Messeinheit 1, aus einer Auswerteeinheit 2, aus einer Ausgabeeinheit 3 und aus einem Gestell 4 für die Aufmachungseinheiten mit dem Prüfgut P, das sind beispielsweise Garn- oder Vorgarnspulen. Gleichmässigkeitsprüfer dieser Art sind bekannt und werden beispielsweise von der Anmelderin der vorliegenden Patentanmeldung unter der Bezeichnung USTER TESTER (USTER eingetragenes Warenzeichen der Zellweger Uster AG) weltweit vertrieben.

Die Messeinheit 1 für das Prüfgut P besteht darstellungsgemäss aus mehreren Modulen, welche in Laufrichtung des Prüfguts P, das ist in der Figur von oben nach unten, wie folgt angeordnet sind: Zuerst ein Modul 5 mit einer Fadenführungseinrichtung 6, beispielsweise einer Fadenbremse, anschliessend ein Modul 7 mit einem Messorgan 8, anschliessend ein Modul 9 mit einer Vorschubeinrichtung 10 und anschliessend ein Modul 11 mit einer Absaugdüse 12. Das unterste Modul 11 ist auf einen Sockel 13 aufgesetzt und alle genannten Module 5, 7, 9 und 11 sowie der Sockel 13 sind in einem Rahmen 14 mit einem bügelartigen Oberteil 15 angeordnet und von diesem Rahmen gehalten.

Das Messorgan 8, durch welches das Prüfgut P durch die durch ein Walzenpaar gebildete Vorschubeinrichtung 10 gezogen wird, ist ein sogenanntes kapazitives Messorgan. Dieses ist in den US Patenten 3 754 172, 3 788 138 und 3 805 607 beschrieben, auf deren Offenbarung hiermit Bezug genommen wird. Die Absaugdüse 12 ist von dem bereits genannten USTER TESTER bekannt und wird hier nicht näher beschrieben.

Die Auswerteeinheit 2 enthält unter anderem einen Analog-/Digitalwandler und einen Rechner und ist darstellungsgemäss mit einem Bildschirm kombiniert. Die vom Messorgan 8 laufend erzeugten elektrischen Signale werden vom Rechner der Auswerteeinheit 2 verarbeitet und in geeigneter Form in einem in der Auswerteeinheit 2 integrierten Speicher gespeichert und können vor ihrem Ausdruck auf dem Ausgabeeinheit 3 bildenden Drucker auf dem Bildschirm dargestellt werden. Dies hat der Vorteil, dass man alle anfallenden Daten vorerst auf dem Bildschirm zur Anzeige bringen und nur ausgewählte Daten für den Ausdruck auf dem Drucker 3 bestimmen kann.

Es sei noch darauf hingewiesen, dass die Signalverarbeitung in der Auswerteeinheit 2 aus drei Hauptkomponenten besteht, nämlich aus dem Spektrographen, für das sogenannte Spektrogramm (Wellenlängenspektrum der Masseschwankungen), aus dem Imperfection Indicator, welcher Grenzwertüberschreitungen der Masse zählt, und aus dem eigentlichen Auswerteteil für die Bestimmung des sogenannten Variationskoeffizienten und der Längenvariationskurve. Alle diese Kenngrössen sind von dem schon genannten USTER TESTER her bekannt.

Wenn mit dem dargestellten Gleichmässigkeitsprüfer die Masseschwankungen von Filamentgarnen bestimmt werden sollen, dann muss eine Messeinheit 1 mit einem anderen Aufbau verwendet werden, welche gegenüber der in der Figur dargestellten Messeinheit 1 folgende Abweichungen aufweist:
- Es wird ein anderes Messorgan 8 benötigt, welches aber ebenfalls ein kapazitives Messorgan ist.
- Die Vorschubeinrichtung 10 muss in Laufrichtung des Prüfguts P vor dem Messorgan 8 angeordnet sein.
- Es wird eine spezielle Absaugdüse 12 benötigt, welche dem Filamentgarn den für die Prüfung erforderlichen Drall verleiht. Bezüglich dieser Absaugdüse wird hiermit auf das US-Patent Nr. 3 951 321 verwiesen.

Durch Einfügen weiterer Module in die Messeinheit 1 kann diese für die Bestimmung weiterer Parameter des Prüfguts P ausgebaut werden. So kann beispielsweise mit einem zusätzlichen Modul mit einem Messorgan für die Haarigkeit des Prüfguts P zusätzlich zu den Masseschwankungen und in einem einzigen Durchlauf des Prüfguts P durch die Messeinheit 1, dessen Haarigkeit bestimmt werden. Bezüglich dieser Ausbaumöglichkeit der Messeinheit 1 wird auf das deutsche Gebrauchsmuster 8708187 der Anmelderin der vorliegenden Patentanmeldung verwiesen.

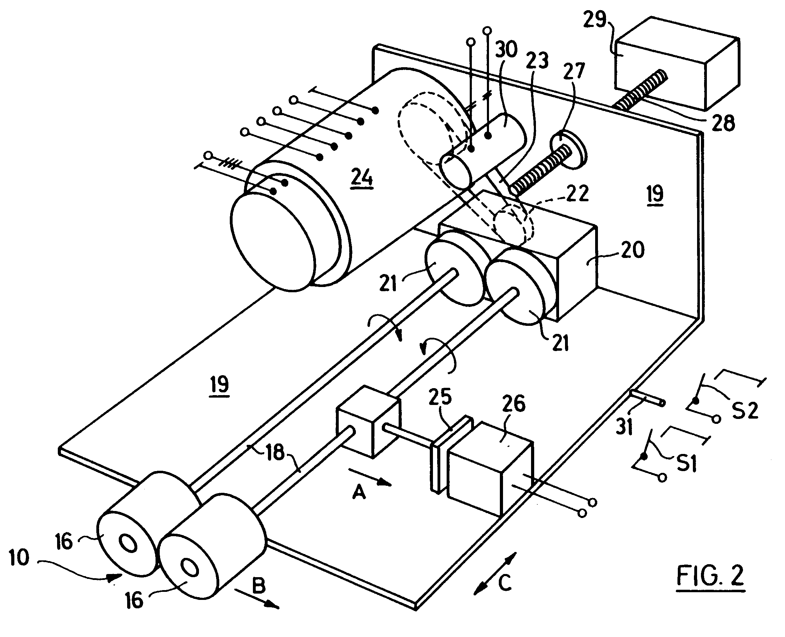

Fig. 2 zeigt eine perspektivische Prinzipdarstellung der wesentlichen Teile der Vorschubeinrichtung 10 der Messeinheit 1 des Gleichmässigkeitsprüfers von Fig. 1. Diese ist darstellungsgemäss durch ein Paar von Transportwalzen 16 aus einem hartgummiähnlichen Material gebildet, welche an der Stirnseite von zwei motorisch antreibbaren Wellen 18 montiert sind. Die Wellen 18 sind in einem auf einer L-förmigen Trägerplatte 19 montierten Lagerblock 20 gelagert und tragen je ein Zahnrad 21. Die beiden Zahnräder 21 stehen einerseits in gegenseitigem Eingriff und sind anderseits mit einer Riemenscheibe 22 verbunden, welche über einen Zahnriemen 23 mit einem Antriebsmotor 24 verbunden ist. Somit sind also beide Transportwalzen 16 formschlüssig mit dem Antriebsmotor 24 verbunden.

Die beiden Transportwalzen 16 berühren im Ruhestand einander; im Betrieb ist ihr gegenseitiger Abstand von der Dicke des Prüfguts abhängig. Das Einlegen des Prüfguts in die Messeinheit 1 (Fig. 1) erfolgt durch einen Einlegearm (nicht dargestellt), welcher das Prüfgut P ergreift und in die Fadenführungseinrichtung 6, in das Messorgan 8 und zwischen die Transportwalzen 16 einlegt und es ausserdem der Absaugdüse 12 (Fig. 1) vorlegt. Zur Erleichterung des automatischen Einlegens des Prüfguts zwischen die Transportwalzen 16 sind diese voneinander wegschwenkbar ausgebildet.

Zu diesem Zweck ist an einer der beiden Wellen 18, darstellungsgemäss an der rechten, ein stempelartiges Verstellorgan 25 befestigt, welchem ein Elektromagnet 26 zugeordnet ist und welches bei Erregung des Elektromagneten 26 von diesem angezogen wird. Dadurch wird die Welle 18 mit dem Verstellorgan 25 in Richtung des Pfeiles A bewegt und die rechte Transportwalwalze 16 wird entsprechend in Richtung des Pfeiles B von der linken Transportwalze weggeschwenkt, wodurch zwischen den beiden Transportwalzen ein Spalt zum Einlegen des Prüfguts gebildet wird. Damit dabei die Zahnräder 21 nicht ausser Eingriff gelangen, ist zumindest die Welle 18 mit dem Verstellorgan 25, vorzugsweise jedoch beide Wellen 18 über einen Teil ihrer Länge als biegsame Welle ausgeführt. Selbstverständlich könnte anstelle der rechten auch die linke Welle 18, oder es könnten auch beide Wellen 18 verschwenkbar ausgebildet sein.

An der Rückwand der L-förmigen Trägerplatte 19 ist ein Gewindeschloss 27 drehbar gelagert, welches in Eingriff mit einer Gewindespindel 28 steht. Die letztere ist an ihrem einen Ende an einem in der Messeinheit 1 (Fig. 1) fest verankerten Bauteil 29 gehalten. Das Gewindeschloss 27 ist mit einem Antriebsmotor 30 verbunden und wird bei dessen Einschalten in Rotation versetzt. Je nach Drehrichtung bewegt sich dann die Trägerplatte 19 mit allen auf ihr befestigten und von ihr getragenen Bauteilen in Richtung des Doppelpfeiles C relativ zum Bauteil 29. Insbesondere vollführen die Transportwalzen 16 eine Hubbewegung in Richtung der Längsachsen der Wellen 18.

Die Trägerplatte 19 ist in einem Gehäuse angeordnet, welches das Modul 9 (Fig. 1) enthält, und die beiden Transportwalzen 16 ragen aus der Frontplatte dieses Gehäuses. Da das Prüfgut P zwischen der Fadenführungseinrichtung 6 und der Absaugdüse 12 (Fig. 1 ) gespannt ist, liegt es an einem definierten Punkt zwischen den beiden einander am nächsten liegenden Erzeugenden am Mantel der beiden Transportwalzen 16. Wenn nun die beiden Transportwalzen 16 die genannte Hubbewegung in Richtung des Pfeiles C ausführen, dann gleitet das Prüfgut entlang dieser Erzeugenden und vollführt relativ zu den Transportwalzen eine Changierbewegung. Der Fadenlauf verändert sich dadurch während des Messvorgangs nicht.

Im Betrieb der Messeinheit 1 (Fig. 1) sind die beiden Antriebsmotoren 24 und 30 ständig angetrieben, und die Transportwalzen 16 sind einerseits rotierend und anderseits in Hubrichtung (Pfeil C) angetrieben. Zur Begrenzung der Hubbewegung der Trägerplatte 19 und damit der Transportwalzen 16 ist an der Trägerplatte 19 ein seitlich abstehendes Betätigungsorgan 31 vorgesehen, in dessen Bewegungsbahn zwei Endschalter S1 und S2 angeordnet sind. Wenn nun das Gewindeschloss 27 so angetrieben wird, dass sich die Trägerplatte 19 und die Transportwalzen 16 aus der gezeichneten Lage nach vorne bewegen, dann kontaktiert das Betätigungsorgan 31 nach einem bestimmten Verstellweg den Schalter S1, durch dessen Betätigung die Drehrichtung des Antriebsmotors umgekehrt wird und Trägerplatte 19 und Transportwalzen 16 in der Gegenrichtung, also nach hinten, verstellt werden. Dies erfolgt bis zum Betätigen des Endschalters S2 durch das Betätigungsorgan 31, wodurch wiederum die Drehrichtung des Antriebsmotors 30 umgekehrt wird, und so weiter.

Dadurch erfolgt die Hubbewegung der Transportwalzen 16 und die Changierbewegung des Prüfguts ständig und vollautomatisch, wobei die Anordnung so ausgelegt ist, dass ein voller Hub, also eine Hin- und Herbewegung der Transportwalzen 16 mit einer Hublänge von etwa 25 mm pro halbem Hub in etwa vier Minuten erfolgt.

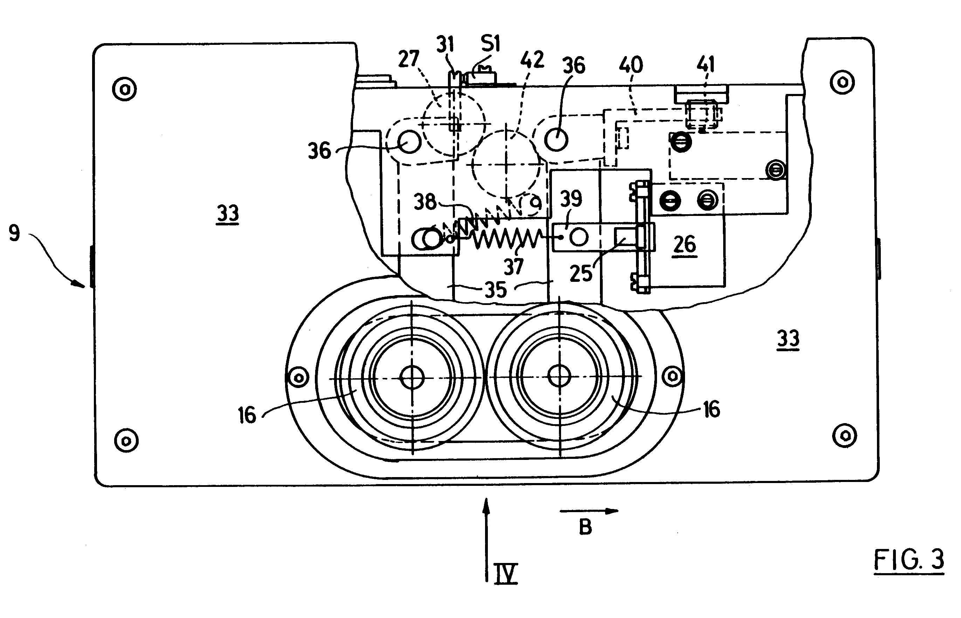

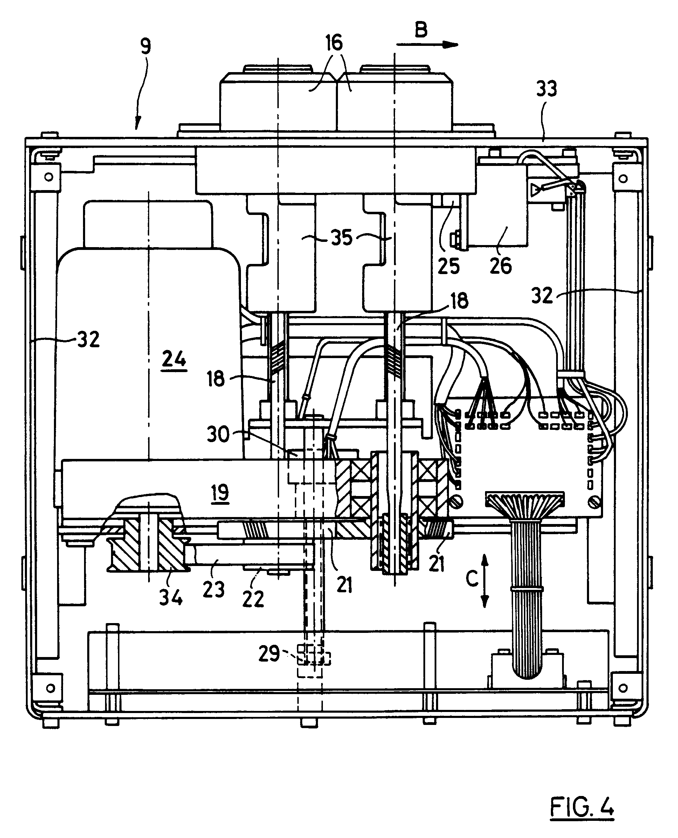

Aus den Fig. 3 und 4 ist die tatsächliche Anordnung der in Fig. 2 schematisch dargestellten Teile im Modul 9 (Fig. 1) ersichtlich. Das letztere besteht darstellungsgemäss aus einem annähernd prismatischen Gehäuse 32, welches in den Rahmen 14 (Fig. 1) eingeschoben ist und aus dessen Frontplatte 33 die beiden Transportwalzen 16 ragen.

Die Trägerplatte 19 ist als zur Frontplatte 33 parallel angeordnete, U-förmige Profilplatte ausgebildet, welche an den beiden Seitenwänden des Gehäuses 32 verschiebbar gelagert ist. Die biegsamen Wellen 18 sind in der Trägerplatte 19 gelagert und tragen an ihrem aus der Rückseite der Trägerplatte 19 ragenden Ende die beiden miteinander in Eingriff stehenden Zahnräder 21. Eine der beiden Wellen, darstellungsgemäss die rechte, trägt die Zahnriemenscheibe 22, welche über den Zahnriemen 23 von der Riemenscheibe 34 des ebenfalls auf der Trägerscheibe 19 befestigten Antriebsmotors 24 angetrieben ist.

Die beiden biegsamen Wellen 18 sind im Bereich der Frontplatte 33 je in einem Lagerarm 35 gelagert, der im Gehäuse 32 vertikal angeordnet ist. Jeder Lagerarm 35 ist an seinem oberen Ende auf einer parallel zu den Wellen 18 angeordneten Lagerwelle 36 schwenkbar gelagert und trägt an seinem unteren Ende die jeweilige Welle 18. Beide Lagerarme 35 sind je durch eine Zugfeder 37, 38 gegeneinander gespannt. Der linke Lagerarm 35 kann gegen die Kraft seiner Zugfeder 38 von Hand nach links geschwenkt werden, was bei besonders dickem Prüfgut erforderlich ist. Am rechten Lagerarm 35 ist eine Lasche 39 befestigt, welche das Verstellorgan 25 (Fig. 2) trägt. Diesem ist der Elektromagnet 26 zugeordnet, bei dessen Erregung das Verstellorgan 25 angezogen und die rechte Transportwalze 16 von der linken weggeschwenkt wird. Dies erfolgt automatisch beim Einlegen des Prüfguts P in die Messeinheit 1 (Fig. 1).

Am oberen Ende des automatisch verschwenkbaren rechten Lagerarm 35 ist ein Finger 40 befestigt, welcher bei aneinander anliegenden Transportwalzen 16, also im Prüfbetrieb, auf einen Mikroschalter 41 drückt. Sobald der den Finger 40 tragende Lagerarm 35 weggeschwenkt wird, gibt der Finger 40 den Mikroschalter 41 frei. Der Mikroschalter 41 ist ein Sicherheitsschalter, der beim Wegschwenken der rechten Transportwalze 16 im Betrieb die Antriebsmotoren 24 und 30 abschaltet. Dieses Wegschwenken im Betrieb tritt dann auf, wenn es bei nicht beaufsichtigter Messeinheit 1 und schwach eingestellter Absaugdüse 12 zu einem mehrfachen Ueberwickeln des Prüfguts auf den Transportwalzen kommt.

An der Trägerplatte 19 ist ausserdem etwa im Niveau der Lagerwellen 36 der Antriebsmotor 30 befestigt, welcher über ein Zahnrad 42 das Gewindeschloss 27 antreibt und dadurch die Hubbewegung der Trägerplatte 19 mitsamt den an dieser befestigten Bauteilen bewirkt. Die beiden im Gehäuse 32 ortfest angeordneten Endschalter S1 und S2 und deren Betätigungsorgan 31 sind aus Fig. 3 ersichtlich.

Wenn auch bei dem dargestellten Ausführungsbeispiel die Hubbewegung der Transportwalzen 16 motorisch und das Oeffnen des Spalts zwischen diesen mittels eines Elektromagneten erfolgt, so sind diese Bewegungen nicht auf die beschriebenen Antriebsarten beschränkt und es könnten selbstverständlich auch hydraulische oder pneumatische Antriebe verwendet werden. Besonders pneumatische Antriebe sind in der Textilindustrie verbreitet, so dass deren Anwendung für den Fachmann naheliegend ist.

## Patentansprüche

1. Vorrichtung zur automatischen Bestimmung von Kenngrössen von textilem Prüfgut (P), wie Garnen, Vorgarnen und Bändern, mit einer eine Führungseinrichtung (6), ein Messorgan (8), eine Vorschubeinrichtung (10) und ein Abzugsorgan (12) für das Prüfgut enthaltenden Messeinheit (1), deren Vorschubeinrichtung durch ein Paar von Transportwalzen (16) gebildet ist, längs deren Achsen beim Durchlaufen des Prüfguts (P) eine Changierbewegung zwischen dem Prüfgut und den Transportwalzen erfolgt, dadurch gekennzeichnet, dass die Transportwalzen (16) mit einem in deren Achsrichtung changierend bewegbaren Verstellorgan (19) verbunden sind.

2. Vorrichtung nach Anspruch 1, dadurch gekennzeichnet, dass das Verstellorgan (19) als kulissenartiger Träger für die Transportwalzen (16) oder für die diese tragenden Wellen (18) ausgebildet und an einen Antrieb (27, 28, 30) angeschlossen ist.

3. Vorrichtung nach Anspruch 2, gekennzeichnet durch Steuermittel (31, S1, S2) zur Umkehr der Bewegungsrichtung der Transportwalzen (16) an den Umkehrpunkten ihrer Changierbewegung.

4. Vorrichtung nach Anspruch 3, dadurch gekennzeichnet, dass die Steuermittel durch entlang des Verstellwegs des Verstellorgans (19) angeordnete, von diesem betätigbare und mit dem Antrieb (27, 28, 30) verbundene Schalter (S1, S2) gebildet sind, bei deren Betätigung eine Umkehrung der Antriebsrichtung erfolgt.

5. Vorrichtung nach Anspruch 4, dadurch gekennzeichnet, dass der Antrieb des Verstellorgans (19) durch einen Motor (30) und durch ein an diesen angeschlossenes Getriebe (27, 28) gebildet ist, und dass die Schalter (S1, S2) mit dem Motor verbunden sind.

## Claims

1. Apparatus for the automatic determination of characteristic magnitudes of textile material (P) to be tested, such as yarns, rovings and slivers, having a measuring unit (1) comprising a guide device (6), a measuring instrument (8), a feed device (10) and a draw-off device (12) for the test material, the feed device being formed by a pair of transport rollers (16) along the axes of which a traverse motion takes place between the test material and the transport rollers at the passage of the test material (P), characterized in that the transport rollers (16) are connected to a displacement device (19) being movable to and fro along the axial direction of the transport rollers.

2. Apparatus according to claim 1, characterized in that the displacement device (19) is formed as a slideably displaceable carrier for the transport rollers (16) or for the shafts (18) carrying the rollers and is connected to a drive (27, 28, 30).

3. Apparatus according to claim 2, characterized by control means (31, S1, S2) for reversing the direction of movement of the transport rollers (16) at the reversal points of their reciprocating movement.

4. Apparatus according to claim 3, characterized in that the control means are formed by switches (S1, S2) arranged along the path of displacement of the displacement device (19) and designed to be operated by this device and connected with the drive (27, 28, 30), the driving direction being reversed when the switches are actuated.

5. Apparatus according to claim 4, characterized in that the drive of the displacement device (19) is provided by a motor (30) and a gear unit (27, 28) connected thereto and in that the switches (S1, S2) are connected to the motor.

## Revendications

1. Dispositif pour la détermination automatique de caractéristiques d'un produit textile (P), tel que des filés, des mèches de préparation et des bandes, avec une unité de mesure (1) comprenant un dispositif de guidage (6), un organe de mesure (8), un dispositif d'avancement (10) et un organe de déroulement (12) du produit textile, dont le dispositif d'avancement est constitué d'une paire de cylindres transporteurs (16) le long des axes desquels, lors du passage du produit textile (P), il se produit un déplacement en va-et-vient entre le produit textile et les cylindres transporteurs, caractérisé en ce que les cylindres transporteurs (16) sont reliés à un organe de réglage (19) pouvant effectuer un déplacement en va-et-vient dans la direction axiale de ceux-ci.

2. Dispositif selon la revendication 1, caractérisé en ce que l'organe de réglage (19) est réalisé sous forme de support à coulisse pour les cylindres transporteurs (16) ou les arbres (18) supportant ceux-ci et est relié à un organe d'entraînement (27, 28, 30).

3. Dispositif selon la revendication 2, caractérisé par des moyens de commande (31, S1, S2) pour inverser la direction de déplacement des cylindres transporteurs (16) aux points de renversement de leur déplacement en va-et-vient.

4. Dispositif selon la revendication 3, caractérisé en ce que les moyens de commande sont constitués par des commutateurs (S1, S2) disposés le long du trajet de déplacement de l'organe de réglage (19), pouvant être actionnés par celui-ci et reliés à l'organe d'entraînement (27, 28, 30), et lors de l'actionnement desquels il se produit un renversement de la direction d'entraînement.

5. Dispositif selon la revendication 4, caractérisé en ce que l'organe d'entraînement de l'organe de réglage (19) est constitué par un moteur (30) et par un engrenage (27, 28) relié à celui-ci, et en ce que les commutateurs (S1, S2) sont reliés au moteur.
